(19) Europäisches Patentamt
European Patent Office
Office européen des brevets

(11) Publication number: 0 347 116 B1

(12) **EUROPEAN PATENT SPECIFICATION**

(45) Date of publication of patent specification: **26.01.94**

(51) Int. Cl.[5]: **C07C 25/18**, C07C 17/12, C08K 5/03

(21) Application number: **89305841.2**

(22) Date of filing: **09.06.89**

(54) **Process for preparing decabromodiphenylalkanes and decabromodiphenylethane.**

(30) Priority: **13.06.88 US 205728**

(43) Date of publication of application: **20.12.89 Bulletin 89/51**

(45) Publication of the grant of the patent: **26.01.94 Bulletin 94/04**

(84) Designated Contracting States: **DE FR GB IT**

(56) References cited:
**DE-A- 2 148 276**
**DE-A- 2 400 455**
**DE-A- 3 422 673**
**US-A- 3 833 674**

**PATENT ABSTRACTS OF JAPAN, vol. 10, no.13 (C-323)(2070), 18 January 1986; & JP-A-60 166 333**

(73) Proprietor: **ETHYL CORPORATION**
**Ethyl Tower**
**451 Florida Boulevard**
**Baton Rouge Louisiana 70801(US)**

(72) Inventor: **Hussain, Saadat**
**5321 Highland Ridge Drive**
**Baton Rouge Louisiana 70817(US)**

(74) Representative: **Collier, Jeremy Austin Grey et al**
**J.A.Kemp & Co.**
**14, South Square**
**Gray's Inn**
**London WC1R 5EU (GB)**

EP 0 347 116 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a process for preparing a product predominant in decabromodiphenylalkane.

Polybromodiphenylalkanes, e.g., decabromodiphenylethane, are known flame retardants for use in polyolefin and in polystyrenic-based formulations. On a commercial basis, the polybromodiphenylalkane would be supplied to the formulation as a product predominant in the polybromodiphenylalkane selected. The product would have a form and an impurity content which would be characteristic of the process used to produce it. If the product's physical characteristics, e.g., thermal stability, limit the formulation's processability, then the processor's desire for the product is limited at best. If the product's color is not white or at least near white, the product will be suitable for use in some formulations, however, the product's use will not be acceptable in formulations calling for a white color.

Therefore, it is an object of this invention to provide a process for producing a product predominant in decabromodiphenylalkane which has good physical characteristics. It is also an object of this invention to provide a process for producing a product which not only has good physical characteristics but which also exhibits at least a near-white color. It is a further object of this invention to provide a formulation containing such decabromodiphenylalkane products. A still further object of this invention is to provide articles made from such formulations.

US 3,833,674 discloses a method of polybrominating biphenyl comprising reacting biphenyl with bromine in the presence of a metal halide halogenation catalyst in solvent quantities of methylene bromide and subsequently adding a lower alkanol to precipitate the polybrominated biphenyl. The method is said to be applicable to at least some other aromatic compounds. The method uses large volumes of methylene bromide solvent and a slight molar excess of bromine. The examples show this method produces a predominance of octo- and nono-brominated biphenyl.

This invention relates to a process for preparing a product which is predominant in decabromodiphenylalkane. The product has good physical and color characteristics and is a flame retardant in formulation with normally flammable macromolecular materials.

The process comprises: forming a stirrable reaction mass by feeding a solution containing methylene bromide and an alpha-omega diphenylalkane to a reaction vessel containing elemental bromine ($Br_2$) and a bromination catalyst; maintaining the reaction mass for a period of time sufficient to achieve perbromination of substantially all of the diphenylalkane; and recovering from the reaction mass the product which is predominant in decabromodiphenyl-alkane.

The diphenylalkane solute portion of the feed solution can be represented by the formula:

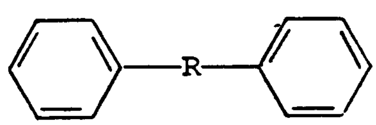

wherein R is an alkylene group containing 1 to 10 carbon atoms. Preferred R groups are methylene and ethylene, which give the preferred reactants, diphenylmethane and 1,2-diphenylethane. Exemplary of other diphenylalkanes are: 1-methyl-1,2-diphenylethane, 1,4-diphenylbutane, 1,6-diphenylhexane, 2,3-dimethyl-1,4-diphenylbutane, 2-ethyl-3-methyl-1,4-diphenylbutane, 2-methyl-1,6-diphenylhexane, 1,9-diphenylnonane and 1,10-diphenyldecane. The diphenylalkane reactant can be produced by various routes. For example, CA 97 38651d (Japanese Kokai 82/45114) and CA 46 7084g disclose the reaction of benzene and ethylene dihalide in the presence of aluminum trichloride to yield diphenylethane. Another process for producing diphenylalkane includes the oxidative dimerization of toluene at a temperature of at least 400°C in the presence of a metal oxide catalyst to yield diphenylethane and diphenylalkene. The latter product is then hydrogenated to remove the olefinic unsaturation.

It is not uncommon for the diphenylalkane reactant to be accompanied by various impurities, especially isomeric impurities. These impurities often give the final decabromodiphenylalkane product an off color. Exemplary of these color-causing impurities are stilbene and 1,1-diphenylethane which often accompany 1,2-diphenylethane. Diminishing the impurity content can be accomplished in a conventional manner, for example, the diphenylalkane can be recrystallized. See Example VII wherein a recrystallization method is described.

The use of methylene bromide as a solvent portion of the feed solution is an important aspect of this invention, as its use gives a superior product. In Table I, which follows the Examples herein, the superior thermostability of a diphenylethane product of this invention is shown.

The feed solution contains from 40 to 85 wt. % methylene bromide. The use of much less methylene bromide, say about 35 wt. %, would yield a reaction mass which is difficult to stir unless a larger excess of $Br_2$ is used than is hereinafter specified. When methylene bromide quantities exceed the upper end of the above range, then process economics are adversely affected as the unneeded extra methylene bromide is an additional cost borne by the process. A preferred amount of methylene bromide is within the range of from 55 to 80 wt. %.

Commercial grades of $Br_2$ may be used in the process of this invention. Should the $Br_2$ contain impurities that would give the final product an off-color, then either the $Br_2$ must be treated to reduce its impurity content or the off-color product must be treated to improve its color. The $Br_2$ is conveniently treated by simple distillation techniques. The off-color product can be treated by washing it with an organic wash solvent after the product is recovered from the reaction mass but prior to its being oven aged. The organic wash solvent can be, e.g., methylene bromide, ethylene dichloride, carbon tetrachloride, xylene, toluene, benzene, acetone, or methanol. Methylene bromide is preferred since it is the same compound used as the solvent in the process.

The catalyst used in the process of this invention is preferably $AlCl_3$ and/or $AlBr_3$, although use may be made of aluminum powder, iron powder, $FeCl_3$ and/or $FeBr_3$, alone or in combination with the aluminum trihalide(s). Other bromination catalysts are suitable provided that they have sufficient catalytic activity to provide for perbromination under the process conditions which will be encountered. Catalytic quantities are used. Typically, catalysts will be present in an amount within the range of 0.1 to 20 wt. % based on the weight of the diphenylalkane reactant used in the process. A preferred amount is within the range of from 1 to 10 wt. % on the same basis.

During the addition of the feed solution to the reaction vessel, the temperature of the reaction mass should be at least about 5 °C. A preferred temperature range is from 10 °C to 80 °C. Most conveniently, the reaction mass can initially be at room temperature and under an atmospheric process pressure. If it is desired to achieve a reaction mass temperature above 50 °C, then provision should be made to prevent or reduce $Br_2$ and/or methylene bromide losses from the reaction mass. This can be achieved by increasing the process pressure to prevent boiling or by providing for refluxing of volatilized $Br_2$ and/or methylene bromide as required.

The feed solution addition will generally occur over a period of time and the addition rate is dependent upon the scale of the reaction and the ability to control the temperature and to handle hydrogen bromide evolution. On a laboratory scale, the addition typically requires 0.5 to 1.5 hours while on a commercial scale, the addition could involve 1.0 to 10.0 hours or longer. Four to five hours would be typical for the commercial scale.

After the addition is complete, the reaction mass is maintained for at least the period of time needed to insure substantial perbromination of the diphenylalkane in high yields. By perbromination it is meant that the aromatic (i.e. phenyl) constituents of the diphenylalkane are brominated to provide a decabromodiphenylalkane. It is preferred that, during this maintenance, the reaction mass temperature be above about 50 °C to minimize maintenance time. While such elevated temperatures are useful, care must be taken not to unduly diminish the $Br_2$ and/or methylene bromide content of the reaction mass by irretrievably boiling the $Br_2$ and/or the methylene bromide from the reaction mass. To prevent such diminishment, the process temperature and pressure can be coordinated so that the $Br_2$ and methylene bromide boiling points are not reached. A more preferred technique is to allow a boiling condition and to provide for refluxing of the evolving vapors back to the reaction mass. Under a preferred pressure, which is about atmospheric, the temperature, at reflux, will be within the range of from 60 °C to 80 °C. The maintenance time at a temperature within this range will be at least about 4 hours.

The amount of $Br_2$ initially present in the reaction vessel should provide at least a 50% excess above the stoichiometric amount needed to produce the desired decabromodiphenylethane product. The temperature of the reaction mass during the maintenance period will influence the amount of $Br_2$ that is preferably used as a larger excess is beneficial at the lower maintenance temperatures while a smaller excess is sufficient at higher maintenance temperatures. For example, at a maintenance temperature of about 50 °C, the excess should be about 150%, while, at maintenance temperature of about 80 °C, the excess can be about 100%. Amounts of $Br_2$ much less than 50% excess will result, at the lowest maintenance temperatures, in a probability of obtaining an under-brominated product. The stoichiometric amount of $Br_2$ is defined as that amount of $Br_2$ needed to provide one Br atom for each substitution site and one Br atom for the attendant formation of HBr which accompanies such substitution. Thus, the stoichiometric amount provides one mole of $Br_2$ per substitution site. By example, decabromodiphenylethane formation will, stoichiometrically, require 10 moles of $Br_2$ to achieve the desired substitution.

After the maintenance period is complete, the product which is predominant in decabromodiphenylalkane is recovered from the reaction mass. Recovery can be by any conventional technique. Preferably, the reaction mass is allowed to cool to room temperature. Water is then added to deactivate the catalyst. Subsequently, the reaction mass is heated to drive off the bromine and methylene bromide still present. After such has been achieved, the product is filtered from the reaction mass and is washed with water to reduce the presence of deactivated catalyst and other impurities. After solvent washing, the product is dried at a temperature of from 140 °C to 170 °C for one hour. The product is then stored or immediately oven aged to further remove entrained $Br_2$, solvent and other impurities. The oven aging generally will occur at a temperature in excess of 150 °C, but below 250 °C, for a period of 4 to 20 hours.

The decabromodiphenylalkane product of this invention, as before noted, has good color and physical characteristics. The product, after oven aging, exhibits high purity and high thermal stability -- indeed, the decabromodiphenylethane product of this invention exhibits a melting point within the range of from 344 °C to 355 °C and a weight loss less than about 20 wt. % at 400 °C when subjected to thermogravimetric analysis and has a weight loss profile as follows,

| | 300 °C | 350 °C | 400 °C |
|---|---|---|---|
| Percent Weight Loss | < 0.5 | < 4.5 | <20.0 |

The color of the decabromodiphenylalkane product of this invention is at least near-white. For example, the decabromodiphenylethane product can have the following Hunter Colorometer values, L = 94 to 95, a = 0 to 0.3, b = 5 to 6 and Y.I. = 10 to 12.

The decabromodiphenylalkane predominant product of this invention may be used as a flame retardant in formulation with virtually any flammable material. The material may be macromolecular, for example, a cellulosic material or a polymer. Illustrative polymers are: olefin polymers, cross-linked and otherwise, for example, homopolymers of ethylene, propylene, and butylene; copolymers of two or more of such alkylene monomers and copolymers of one or more of such alkylene monomers and any other copolymerizable monomers, for example, ethylene/propylene copolymers, ethylene/ethyl acrylate copolymers and ethylene/vinyl acetate copolymers; polymers of olefinically unsaturated monomers, for example, polystyrene, high impact polystyrene, and styrene copolymers; polyurethanes; polyamides; polyimides; polycarbonates; polyethers; acrylic resins; polyesters, especially poly(ethyleneterephthalate) and poly-(butyleneterephthalate); epoxy resins; alkyds; phenolics; elastomers, for example, butadiene/ styrene copolymers and butadiene/acrylonitrile copolymers; terpolymers of acrylonitrile, butadiene and styrene; natural rubber; butyl rubber; and polysiloxanes. The polymer may also be a blend of various polymers. Further, the polymer may be, where appropriate, cross-linked by chemical means or by irradiation.

The amount of the product of this invention used in a formulation will be that quantity needed to obtain the flame retardancy sought. It will be apparent to those skilled in the art that for all cases no single precise value for the proportion of the product in the formulation can be given, since this proportion will vary with the particular flammable material, the presence of other additives and the degree of flame retardancy sought in any given application. Further, the proportion necessary to achieve a given flame retardancy in a particular formulation will depend upon the shape of the article into which the formulation is to be made, for example, electrical insulation, tubing and film will each behave differently. In general, however, the formulation may contain from 5 to 40 wt. %, preferably 10 to 30 wt. %, of the product when it is the only flame retardant compound in the formulation.

It is especially advantageous to use the product of this invention with an inorganic compound, especially ferric oxide, zinc oxide, zinc borate, the oxide of a Group V element, for example, bismuth, arsenic, phosphorus and especially antimony, in the formulation. Of these compounds, antimony oxide is especially preferred. If such a compound is present in the formulation, the quantity of decabromodiphenylalkane predominant product needed to achieve a given flame-retardancy is accordingly reduced.

Formulations containing a flame retardant system comprised of the product of this invention and the above inorganic compounds may contain up to about 40% by weight of the system, preferably between 20% and 30% by weight.

It is believed that the product of this invention and the inorganic compound will react under the conditions of combustion of a flammable material to form inorganic bromine compounds, e.g., hydrogen bromide and oxybromides, which assist in retarding combustion. The bromine-bearing product of this invention also acts as a flame retardant independently and the proportions of the product and inorganic compound in a flame retardant system are a matter of choice, depending on the material in which the system is to be incorporated and commercial considerations. Generally, the product of this invention and

the inorganic compound are in a weight ratio of from 1:1 to 7:1, and preferably of from 2:1 to 4:1.

The formulations containing the product of this invention may contain any of the additives usually present in such formulations, e.g., plasticizers, antioxidants, fillers, pigments, or UV stabilizers.

Thermoplastic articles formed from formulations containing a thermoplastic polymer and a product of this invention can be produced conventionally, e.g., by injection molding, extrusion molding, compression molding, and the like.

The following examples are presented.

EXAMPLE I (Comparative)

This example is not of the invention but rather is presented for comparative purposes. The process illustrated by this example produces a product predominant in decabromodiphenylethane using bromine as both a reactant and as the sole solvent.

A 3-L resin kettle was equipped with a mechanical stirrer, a thermometer, a temperature controller, an addition funnel wrapped with a heating tape, a heating mantle and a double reflux condenser. The kettle was charged with 2407.0g (15.00 moles) of bromine and 8.9g of anhydrous aluminum chloride. The amount of bromine charged represents an amount which is a 150% excess of the stoichiometric amount needed to perbrominate the diphenylethane reactant.

The addition funnel was charged with ground diphenylethane (111.0g, 0.61 moles) and heated slowly by way of the heating tape to provide molten diphenylethane (55°C-66°C). The molten diphenylethane was added to the reactor over a period of 60 to 75 minutes. The resultant reaction mass during this addition was kept at a temperature from 25°C to 30°C.

After the addition was substantially complete, the reaction mass was stirred and heated to reflux (60°C) for 4.5 hours. After this period, the reaction was deemed complete.

Water (1L) was added to the reaction mass to deactivate the catalyst. Bromine was then distilled off until the vapor obtained had a temperature of 100°C indicating that substantially all of the bromine was removed.

The resultant solid product was filtered, washed once with water and then with 250 mL xylene. This washed product was dried and then oven aged at 200°C for 16 hours, to yield 572.0g (96%) of a product predominant in decabromodiphenylethane. The product had a melting point of 340°C-344°C and a bromine content of 82.7%.

EXAMPLE II (Comparative)

This example is presented for comparative purposes and is not of this invention. The process illustrated by this example produces a product predominant in decabromodiphenylethane by using ethylene dichloride as a solvent in the process.

In a glass reaction vessel was placed 256g (1.6 moles) of bromine, 25 mL of ethylene dichloride and 1.5g of anhydrous aluminum chloride. While stirring, a solution of 14.6g (0.08 moles) of diphenylethane in 25 mL of ethylene dichloride was added at 25°C-35°C over a 2-hour period. The reaction mixture was heated to reflux and refluxed for 6 hours. It was then cooled to room temperature and 150 mL of ethylene dichloride added. Bromine was distilled out along with some ethylene dichloride. The mixture was cooled and another 100 mL of ethylene dichloride was added and bromine was again distilled off until only a trace of bromine color remained. The product was recovered by filtration, washed twice with ethylene dichloride and dried at 100°C for 4 hours to give 76.2g (98.5% yield) of a light orange product predominant in decabromodiphenylethane. The product was oven aged at 200°C for 16 hours to give an off-white material having a melting point of 335°C-342°C and a bromine content of 82.1% (calculated = 82.1%).

Examples III-VI illustrate processes of this invention.

EXAMPLE III

A 500-mL-resin kettle was equipped with a mechanical stirrer, reflux condenser, a thermometer, a temperature controller and an addition funnel.

The kettle was charged with 320g of $Br_2$ (2.0 moles) and 1.8g of aluminum chloride. The 320g of bromine represents a 100% excess of bromine beyond the stoichiometric amount required for perbromination.

The addition funnel was charged with a solution containing 18.2g diphenylethane (0.1 mole) and 49.4g of methylene bromide (20 mL). The solution was added via the funnel dropwise into the kettle over a period

of 30 minutes. During the addition, the resultant reaction mass was stirred at room temperature. After the addition was complete, the reaction mass was stirred and heated to reflux (65°C-67°C) for 6 hours.

After the 6-hour period had lapsed, the reaction was deemed complete and the reaction mass was cooled to room temperature. Water (150 mL) was added to the reaction mass to deactivate the catalyst. After catalyst deactivation the reaction mass was heated to remove bromine and methylene bromide still present until the vapor obtained was approximately 100°C.

The solid product was filtered from the remaining solution and washed twice with 200-mL-portions of water and dried at 160°C for one hour. The product was then oven aged at 200°C in a forced-air oven for 15 hours. A white product (95.5g, 98%) was obtained. The product had a melting point of 344°C-354°C and an average bromine content of 82.6%.

EXAMPLE IV

A resin kettle (3L) was equipped with a mechanical stirrer, reflux condenser, a thermometer with a temperature controller and an addition funnel.

The kettle was charged with 2160g of $Br_2$ (13.5 moles) and 10.9g of anhydrous aluminum chloride. The amount of $Br_2$ used in this example represents an 80% excess of $Br_2$ beyond the stoichiometric amount required for perbromination.

The addition funnel was charged with a solution containing 136.8g diphenylethane (0.75 mole) and 185.2g of methylene bromide (75 mL). The solution was added via the funnel dropwise into the kettle over a period of 95 minutes. During the addition, the resultant reaction mass was stirred at room temperature. After the addition was complete, the reaction mass was stirred and heated to reflux (65°C-66°C) for 5 hours.

After the 5-hour period had lapsed, the reaction was deemed complete and the reaction mass was cooled to room temperature. Water (800 mL) was added to the reaction mass to deactivate the catalyst. After deactivation of the catalyst, the reaction mass was heated to remove bromine and methylene bromide still present. The heat was removed when the vapor was at 100°C.

The solid decabromodiphenylethane predominant product was filtered from the reaction mass and washed twice with 250-mL-portions of water and then twice with 250-mL-portions of xylene. The recovered product was dried in air at room temperature overnight. The product was then oven aged at 200°C in a forced-air oven for 12 hours. A light tan product (719.8g, 98.7%) was obtained. The product had a melting point of 340°C-350°C, and a bromine content of 83.3%.

EXAMPLE V

The procedure of Example III was followed except that 30 mL (74.4g) of methylene bromide was used to dissolve 18.2g of diphenylethane (18.2g) and the recovered product was oven aged for 16 hours at 200°C.

An off-white predominantly decabromodiphenylethane product (93.0g, 95.6%) was obtained. This product had a melting point of 345°C-354°C and a bromine content of 83.4%.

EXAMPLE VI

A 3-L resin kettle was equipped with a mechanical stirrer, reflux condenser, an addition funnel, a thermometer with a temperature controller and a heating mantle. The kettle was charged with 1600g of bromine (10 moles, 100% excess of stoichiometric) and 9.0g of anhydrous aluminum chloride.

The addition funnel was charged with a solution containing 91.0g diphenylethane (0.5 mole) and 185g of methylene bromide (75 mL). The solution was added to the kettle over a period of 40 minutes.

After the addition was complete, another 62.0g of methylene bromide (25 mL) was added to aid in the stirrability of the reaction mass. The reaction was then heated to 66°C-67°C and stirred at that temperature for 6 hours.

After the 6-hour period had lapsed, the reaction mass was cooled to 50°C. Water (500 mL) was added to the reaction mass to deactivate the catalyst. The reaction mass was then heated to remove the $Br_2$ and methylene bromide therefrom. After this removal was effected, the white product was filtered from the reaction mass.

The white product, which was predominantly decabromodiphenylethane, was washed twice with 400-mL portions of water. After this washing, the recovered product was dried at 125°C for 2 hours. After drying, the product was oven aged at 200°C for 8 hours in a forced-air oven.

After the oven aging was effected the product was washed twice with 400-mL portions of toluene and then dried at room temperature. After drying, the product was again heated at 200 °C for another 2 hours to remove any solvent which may be still present.

The final yield of the product was 475.4g (97.8%). The final product was subjected to thermogravimetric analysis and had a 0.0 wt. % loss at 200 °C and 300 °C, a 17.0 wt. % loss at 400 °C and a 84.9 wt. % loss at 500 °C.

The final product was also tested for color with a Hunter Colorometer which gave the following values: L = 91.5, a = 0.3, b = 9.98, and Y.I. = 20.0.

The final product also had a melting point of 344 °C-352 °C.

EXAMPLE VII

A portion of the final product produced in Example VI was formulated with Huntsman 840D HIPS (High Impact Polystyrene) and antimony oxide synergist. The decabromodiphenylethane predominant product was present in an amount of about 12 wt. % while the antimony oxide synergist was present in an amount of about 4 wt. %. All weight percents were based upon the total weight of the formulation.

The formulations were made by extrusion on a Haake System Forty Extruder at 205 °C-225 °C. The recovered formulation was then injection molded on a Battenfeld injection molder at a zone temperature of 200 °C-227 °C.

The injection molded articles were tested in accordance with UL 94 and 1/8-inch strips gave a rating of V-0. Further, the injection molded articles were subjected to tests for determination of UV stability. A $\Delta E_{48}$-(Sun-lighter) of 28.5 was obtained.

EXAMPLE VIII

The following example illustrates a method for purifying diphenylethane reactant to obtain a good color.

A 1-L beaker was charged with methanol (300 mL). Crude diphenylethane (300g) was then added. The contents of the beaker were heated and stirred at 65 °C, and the resulting clear solution was then allowed to cool slowly to room temperature. A crystalline solid was formed. The solid was filtered and washed once with 120 mL methanol and then dried. The recovery was 274.5g (91.5%). The recrystallized material had a melting point of 50 °C-54 °C which is slightly higher than the 49 °C-50 °C for the original starting diphenylethane. The starting diphenylethane had a Yellowness Index of 33.2 (L = 81.2, a = -2.9, b = 16.1) while the recrystallised diphenylethane material had a Yellowness Index of 2.8. (L = 90.8, a = -0.4, b = 1.4).

It is believed that the diphenylethane reactant should have a Yellowness Index of 5 or less as measured by the Hunter Colorometer so that the product predominant in decabromodiphenylethane will have a good color.

The following table illustrates the superior thermal stability of the product of the process of this invention.

TABLE I

THERMOGRAVIMETRIC ANALYSIS

| Sample | Percent Weight Loss at 300°C | 350°C | 400°C | 450°C | 500°C |
|---|---|---|---|---|---|
| From Example I (not of the invention) | 0.8 | 5.3 | 28.3 | 79.6 | 99.6 |
| From Example III (of the invention) | 0.4 | 3.5 | 14.6 | 61.6 | 91.4 |
| From Example IV (of the invention) | 0.4 | 3.1 | 15.9 | 71.3 | 91.5 |

| Sample | Percent Weight Loss at 320°C | 357°C | 375°C | 395°C |
|---|---|---|---|---|
| From Example II (not of the invention) | 2.0 | 5.0 | 10 | 25 |

All thermogravimetric analyses were performed in the presence of air and with a DuPont Model 990 Thermal Analyzer, equipped with a Model 951 TGA Module.

A typical measurement was performed by placing 10-20 mg of the material to be tested in the platinum sample holder, followed by enclosing the sample holder in a quartz tube. The entire assembly was then inserted into the furnace, and the air flow was adjusted to 50 mL/min. The system was programmed to run from 25°C to 500°C at a heating rate of 10°C per minute. The weight loss vs. temperature was recorded automatically by the instrument.

**Claims**

1. A process for preparing a product predominant in decabromodiphenylalkane, which process comprises:
   (a) forming a stirrable reaction mass by adding a solution comprising an alpha-omega diphenylalkane and 40-85 wt. % methylene bromide to a reaction vessel containing $Br_2$ and a bromination catalyst, said bromine being present in an amount which is at least 50% in excess of the stoichiometric amount needed to perbrominate the diphenylalkane;
   (b) maintaining the reaction mass for a period of time sufficient to achieve perbromination of substantially all of said diphenylalkane; and
   (c) recovering said product predominant in decabromodiphenylalkane from said reaction mass.

2. The process of claim 1 wherein said decabromodiphenylalkane is decabromodiphenylethane or decabromodiphenylmethane and said diphenylalkane is, respectively, diphenylethane or diphenylmethane.

3. A decabromodiphenylethane product obtainable by the process of claim 2 containing a predominant amount of decabromodiphenylethane and a lesser amount of impurities, exhibiting, after oven aging at 200°C, a weight loss less than about 20 wt. % at 400°C when subjected to thermogravimetric analysis.

4. A decabromodiphenylethane product according to claim 3 having, after oven aging at 200°C, the following weight loss profile when subjected to thermogravimetric analysis,

| | 300 ° C | 350 ° C | 400 ° C |
|---|---|---|---|
| Percent Weight Loss | < 0.5 | < 4.5 | < 15.5 |

**5.** A decabromodiphenylethane product according to claim 3 or claim 4 said decabromodiphenylethane product having a melting point after oven aging at 200 ° C within the range of from 344 ° C to 355 ° C.

**6.** A thermoplastic formulation containing high impact polystyrene, an acrylonitrile-butadiene-styrene terpolymer, or a polyolefin and a flame retardant amount of the decabromodiphenylethane product of claim 3, 4 or 5.

**Patentansprüche**

**1.** Verfahren zur Herstellung eines im wesentlichen Decabromdiphenylalkan enthaltenden Produktes, bei dem man

a) durch Zugabe einer Lösung, die ein alpha-omega-Diphenylalkan und 40 - 85 Gew. Methylenbromid umfaßt, in ein Reaktionsgefäß, das $Br_2$ und einen Bromierungskatalysator enthält, eine rührbare Reaktionsmasse herstellt, wobei das Brom in einer Menge vorhanden ist, die mindestens 50 % über der stöchiometrischen Menge liegt, die für die Perbromierung des Diphenylalkans erforderlich ist,;

b) die Reaktionsmasse für einen Zeitraum hält, der notwendig ist, um die Perbromierung im wesentlichen des ganzen Diphenylalkans zu erreichen, und

c) das im wesentlichen Decabromdiphenylalkan enthaltende Produkt aus der Reaktionsmasse entnimmt.

**2.** Verfahren nach Anspruch 1, bei dem das Decabromdiphenylalkan Decabromdiphenylethan oder Decabromdiphenylmethan ist und das Diphenylalkan Diphenylethan bzw. Diphenylmethan ist.

**3.** Decabromdiphenylethanprodukt, erhältlich durch das Verfahren nach Anspruch 2, das eine vorherrschende Menge von Decabromdiphenylethan und eine geringere Menge von Verunreinigungen enthält, und beim Altern im Ofen bei 200 ° C einen Gewichtsverlust aufweist, der bei thermogravimetrischer Analyse weniger als etwa 20 Gew.% bei 400 ° C ausmacht.

**4.** Decabromdiphenylethanprodukt nach Anspruch 3, das nach Altern im Ofen bei 200 ° C bei thermogravimetrischer Analyse folgendes Gewichtsverlustprofil aufweist:

| | 300 ° C | 350 ° C | 400 ° C |
|---|---|---|---|
| Prozent Gewichtsverlust | < 0,5 | < 4,5 | < 15,5 |

**5.** Decabromdiphenylethanprodukt nach Anspruch 3 oder 4, das nach dem Altern im Ofen bei 200 ° C einen Schmelzpunkt im Bereich von 344 ° C bis 355 ° C aufweist.

**6.** Thermoplastische Formulierung die hochschlagfestes Polystyrol, ein Acrylonitril-butadien-styrol-Terpolymer oder ein Polyolefin und eine flammhemmende Menge des Decabromdiphenylethanprodukts nach Anspruch 3, 4 oder 5 enthält.

**Revendications**

**1.** Procédé d'obtention d'un produit à prédominance de décabromodiphénylalcane, procédé qui comprend les étapes consistant :

(a) à former un mélange réactionnel pouvant être agité, par addition d'une solution comprenant un alpha-oméga-diphénylalcane et 40 à 85 % en poids de bromure de méthylène à un récipient de réaction contenant du $Br_2$ et un catalyseur de bromation, ledit brome étant présent en une quantité en excès d'au moins 50 % par rapport à la quantité stoechiométrique nécessaire pour la perbromation du diphénylalcane ;

(b) à maintenir le mélange réactionnel pendant un temps suffisant pour réaliser la perbromation de la quasi-totalité du diphénylalcane ; et

(c) à séparer du mélange réactionnel le produit qui prédomine en décabromodiphénylalcane.

**2.** Procédé suivant la revendication 1, dans lequel le décabromodiphénylalcane est le décabromodiphényléthane ou le décabromodiphénylméthane et le diphénylalcane est, respectivement, le diphényléthane ou le diphénylméthane.

**3.** Décabromodiphényléthane obtenu comme produit par le procédé suivant la revendication 2, contenant une quantité prédominante de décabromodiphényléthane et une quantité moins grande d'impuretés, présentant, après vieillissement au four à 200 °C, une perte de poids inférieure à environ 20 % en poids à 400 °C lorsqu'il est soumis à une analyse thermogravimétrique.

**4.** Décabromodiphényléthane suivant la revendication 3 ayant, après vieillissement au four à 200 °C, le profil de perte de poids suivant lorsqu'il est soumis à une analyse thermogravimétrique,

| | 300 °C | 350 °C | 400 °C |
|---|---|---|---|
| Perte de poids, % | < 0,5 | < 4,5 | >15,5 |

**5.** Décabromodiphényléthane suivant la revendication 3 ou la revendication 4, ledit décabromodiphényléthane ayant un point de fusion après vieillissement au four à 200 °C compris dans la plage de 344 °C à 355 °C.

**6.** Formulation thermoplastique contenant du polystyrène de grande résistance au choc, un terpolymère acrylonitrile-butadiène-styrène ou une polyoléfine et une quantité à effet retardateur de flamme de produit qui est le décabromodiphényléthane suivant la revendication 3, 4 ou 5.